# EUROPEAN PATENT APPLICATION

(11) **EP 3 061 424 A1**
(43) Date of publication of application: **31.08.2016**
(21) Application number: 14855508.9
(22) Date of filing: 24.10.2014
(51) Int. Cl.: A61F 2/848, A61F 2/844

(54) **MEDICAL STENT**

(30) Priority: 25.10.2013 US 201361895541 P
(71) Applicant: Olympus Corporation, Hachioji-shi Tokyo 192-8507 (JP)
(72) Inventor: YANUMA, Yutaka, Tokyo 192-8507 (JP)
(74) Representative: Gunzelmann, Rainer
(86) International application number: PCT/JP2014/078351
(87) International publication number: WO 2015/060424

(57) **Abstract**

A medical stent is provided including a longitudinal member which has an internal space that extends along a longitudinal axis and a side opening that is formed on a side surface so as to communicate with the internal space, and allows an insertion member that is inserted into the internal space to move along the longitudinal axis; a flap portion which has an extension portion that extends radially outward from the side surface of the longitudinal member so as to engage with tissue and a fixed portion that is fixed to the longitudinal member, being continuous with the extension portion, and is transformable between an open state in which the extension portion radially extends from the side surface of the longitudinal member and a closed state in which the extension portion extends along the longitudinal axis of the longitudinal member; and a flap radial-expansion prevention portion which at least partially passes through the side opening and allows the insertion member to hold the flap portion in the internal space when the flap portion is in the closed state.

## Description

### Technical Field

The present invention relates to a medical stent which is used by being inserted into a body cavity.

Priority is claimed on United States Patent Application No. 61/895,541, filed October 25, 2013, as provisional application in the United States, the content of which is incorporated herein by reference.

### Background Art

In the related art, placement of a stent is performed with respect to a stenosed part formed in a bile duct in order to expand the stenosed part and to maintain a persistently open state thereof. In the placement of the stent, a method in which a whole portion of the stent is placed in the bile duct so as to prevent a proximal end side of the stent from protruding in a lumen of the duodenum at the time of placing is under examination.

Patent Literature 1 discloses a known stent delivery system which is used in the aforementioned case.

In the stent delivery system, a long flexible insertion portion extends from an operation part operated by an operator, and a stent is detachably mounted at a distal end portion of the insertion portion.

The insertion portion includes a pusher catheter. A guide sheath (insertion member) is inserted into a lumen of the pusher catheter so as to freely advance and retreat. The distal end portion of the pusher catheter is provided with an abutted surface on which a proximal end surface of the stent can abut and a stent accommodation portion in which at least a portion of the stent is accommodated. The stent accommodation portion has a configuration in which the pusher catheter dilates radially outward and the diameter of the lumen is greater than the diameter of the stent.

In the stent, flaps are respectively provided at both end portions of a cylindrical stent main body in the axial direction. The inner diameter of the stent main body is set so as to allow the guide sheath to be inserted through the inside thereof. However, the inner diameter of the distal end portion is slightly decreased. Therefore, the stent can lightly engage with the guide sheath due to friction therebetween.

The flap of the stent on the distal end side is formed so as to open toward the proximal end side in its natural state. The flap on the proximal end side is formed so as to open toward the distal end side in its natural state. When the stent is accommodated in the stent accommodation portion, each of the flaps is closed so as to be folded by being pressed against an inner wall of the stent accommodation portion.

A technical procedure of placing a stent performed by using the stent delivery system having the above configuration will be disclosed hereinafter.

First, an endoscope is inserted to the vicinity of the duodenal papilla through a natural opening such as a mouth of a patient. The insertion portion of the stent delivery system is inserted into an operative channel of the endoscope, and the stent accommodation portion is caused to protrude from a distal end of the endoscope. The guide sheath and the insertion portion are inserted from the papilla in order, and the stent accommodation portion is introduced to a stenosed part formed in the bile duct.

The guide sheath is removed from the stent. When the insertion portion is retracted, the stent accommodation portion is retreated and the stent is exposed. The flaps pressed by the stent accommodation portion are opened, and the stenosed site in the bile duct is positioned between the flaps on the distal end side and the proximal end side. The flaps prevent the stent from moving.

### Citation List

### Patent Literature

[Patent Literature 1] Japanese Unexamined Patent Application, First Publication No. 2009-136676

### Summary of Invention

### Technical Problem

In the stent delivery system disclosed in Patent Literature 1, when a stent is attached to a stent accommodation portion, the stent accommodation portion is attached so as to cover an outer circumferential surface of the stent on a proximal end side. Therefore, the outer diameter of the stent delivery system in its entirety at the time of placement of the stent becomes large so that introduction characteristics with respect to the bile duct and the like are deteriorated.

An ordinary pusher catheter which is used when placing a stent have tubular shapes, and the pusher catheter which is provided with the abutting surface and the stent accommodation portion disclosed in Patent Literature 1 is a special treatment device.

The present invention has been made taking the above-described circumstances into consideration and aims to provide a stent in which a closed state of a flap portion can be maintained while requiring no special treatment device for covering an outer circumferential surface of the stent when introducing the stent into a human body.

### Solution to Problem

According to a first aspect of the present invention, a medical stent includes a longitudinal member which has an internal space that extends along a longitudinal axis and a side opening that is formed on a side surface so as to communicate with the internal space, and allows an insertion member that is inserted into the internal space to move along the longitudinal axis; a flap portion which has an extension portion that extends radially outward from the side surface of the longitudinal member so as to engage with tissue and a fixed portion which is fixed to the longitudinal member, being continuous with the extension portion, and is transformable between an open state in which the extension portion radially extends from the side surface of the longitudinal member and a closed state in which the extension portion extends along the longitudinal axis of the longitudinal member; and a flap radial-expansion prevention portion which at least partially passes through the side opening and allows the insertion member to hold the flap portion in the internal space when the flap portion is in the closed state.

According to a second aspect of the present invention, in the first aspect, the flap radial-expansion prevention portion may be configured to be an annularly formed filamentous member. The side opening may be formed on the flap portion side with respect to the longitudinal axis of the longitudinal member. The flap radial-expansion prevention portion may be held in the internal space by the insertion member when the insertion member is inserted through the annular flap radial-expansion prevention portion.

According to a third aspect of the present invention, in the first aspect, the flap radial-expansion prevention portion may be arranged outside the internal space of the longitudinal member when the flap portion is in the open state.

According to a fourth aspect of the present invention, in the first aspect, the flap radial-expansion prevention portion may be configured to be an annularly formed filamentous member. The side opening may be formed on a side opposite to the flap portion with respect to the longitudinal axis of the longitudinal member. The flap radial-expansion prevention portion may be held in the internal space by the insertion member when the filamentous member passing through the side opening and being arranged along an inner surface of the internal space in a circumferential direction is held so as to prevent the insertion member from moving to the side opening side.

According to a fifth aspect of the present invention, in the first aspect, the flap radial-expansion prevention portion may be a filamentous member. The flap portion may be provided with a pressed portion which is pressed by the filamentous member so that a force for transforming the flap portion from the open state to the closed state is applied to the flap portion.

According to a sixth aspect of the present invention, in the fifth aspect, the extension portion of the flap portion may be provided with an engagement portion so as to prevent positional misalignment of the pressed portion in the flap portion.

According to a seventh aspect of the present invention, in the sixth aspect, the engagement portion may be a penetration hole which is formed in the pressed portion so as to extend in a thickness direction of the flap portion. The filamentous member may be inserted through the penetration hole.

According to an eighth aspect of the present invention, in the sixth aspect, the engagement portion may be a groove portion which is formed at one end of the pressed portion in a width direction of the flap portion so as to penetrate in a thickness direction of the flap portion. The filamentous member may be arranged in the groove portion.

According to a ninth aspect of the present invention, in the first aspect, the longitudinal member may have a tubular shape. The flap portion may be a portion of the side surface of the longitudinal member. The side opening may be an opening which is formed on the side surface of the longitudinal member by cutting and raising the side surface of the longitudinal member.

According to a tenth aspect of the present invention, in the first aspect, the flap radial-expansion prevention portion may be formed to have a belt shape. The flap radial-expansion prevention portion may be held in the internal space by the insertion member due to a frictional force which is applied between the flap radial-expansion prevention portion passing through the side opening and being arranged in the internal space and the insertion member.

According to an eleventh aspect of the present invention, in the first aspect, the flap radial-expansion prevention portion may have a first end portion which is connected to the extension portion of the flap portion and a second end portion which is continuous with the first end portion. The second end portion of the flap radial-expansion prevention portion may at least partially pass through the side opening and the second end portion may engage with the insertion member so as to cause the flap portion to maintain the closed state when the flap portion is in the closed state.

### Advantageous Effects of Invention

According to each of the above-described aspects, it is possible to maintain a closed state of a flap portion while requiring no special treatment device for covering an outer circumferential surface of a stent when introducing the stent into a human body.

### Brief Description of Drawings

FIG. 1 is a diagram schematically illustrating a cross-section of a side surface of a stent, according to a first embodiment of the present invention.
FIG. 2 is a side view of a main portion of the stent according to the first embodiment of the present invention.
FIG. 3 is a perspective view of the cutoff main portion of the stent according to the first embodiment of the present invention.
FIG. 4 is a diagram illustrating a procedure for placing the stent, according to the first embodiment of the present invention.
FIG. 5 is a cross-sectional view of the side surface in a state where a guide sheath is inserted through the stent, according to the first embodiment of the present invention.
FIG. 6 is a cross-sectional view taken along line A1-A1 in FIG. 5.
FIG. 7 is a diagram illustrating the procedure for placing the stent, according to the first embodiment of the present invention.
FIG. 8 is a diagram illustrating the procedure for placing the stent, according to the first embodiment of the present invention.
FIG. 9 is a side view of the main portion of the stent according to a modification example of the embodiment of the present invention.
FIG. 10 is a plan view of the cutoff main portion of the stent according to the modification example of the embodiment of the present invention.
FIG. 11 is a plan view of the main portion of the stent according to the modification example of the embodiment of the present invention.
FIG. 12 is a cross-sectional view taken along line A2-A2 in FIG. 11.
FIG. 13 is a cross-sectional view of the main portion of the stent according to the modification example of the embodiment of the present invention.
FIG. 14 is a plan view of the main portion of the stent according to the modification example of the embodiment of the present invention.
FIG. 15 is a cross-sectional view taken along line A3-A3 in FIG. 14.
FIG. 16 is a plan view of the main portion of the stent according to the modification example of the embodiment of the present invention.
FIG. 17 is a cross-sectional view taken along line A4-A4 in FIG. 16.
FIG. 18 is a side view of the main portion in a state where the guide sheath is inserted through the stent, according to a second embodiment of the present invention.
FIG. 19 is a cross-sectional view taken along line A6-A6 in FIG. 18.
FIG. 20 is a side view of the main portion in a state where the guide sheath is pulled out from the stent, according to the second embodiment of the present invention.
FIG. 21 is a cross-sectional view taken along line A7-A7 in FIG. 20.
FIG. 22 is a side view of the main portion in a state where the guide sheath is inserted through the stent, according to a third embodiment of the present invention.
FIG. 23 is a cross-sectional view taken along line A8-A8 in FIG. 22.
FIG. 24 is a side view of the main portion in a state where the guide sheath is pulled out from the stent, according to the third embodiment of the present invention.
FIG. 25 is a cross-sectional view taken along line A9-A9 in FIG. 24.

### Description of Embodiments

### (First Embodiment)

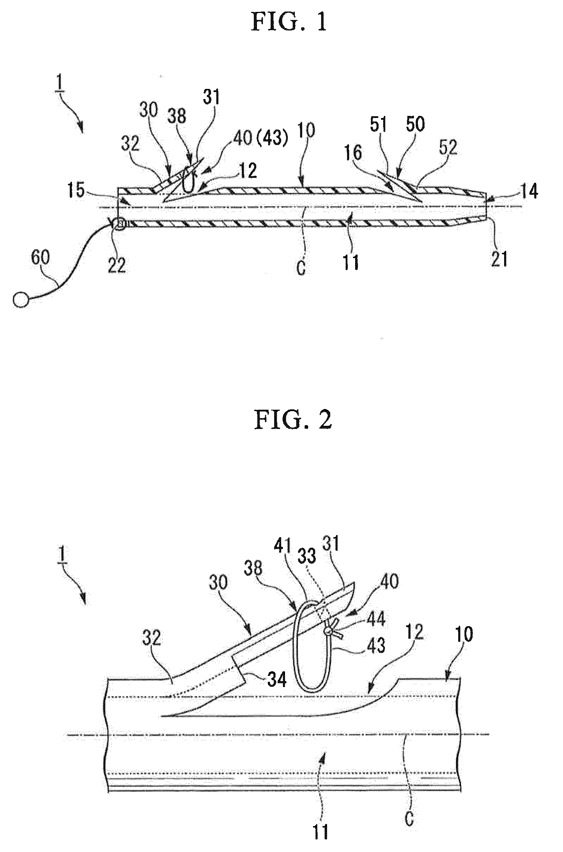

Hereinafter, a first embodiment of a medical stent (hereinafter, also referred to as "stent") according to the present invention will be described with reference to FIGS. 1 to 17. In all the diagrams below, the thicknesses and the ratios between the dimensions of each of the configuration elements are illustrated by being varied in a suitable manner in order to allow the diagrams to be easily viewed.

As illustrated in FIGS. 1 and 2, a stent 1 of the present embodiment includes a main body (a longitudinal member) 10 which has an internal space 11 that extends along a longitudinal axis (the central axis) C, and a side opening 12 that is formed on a side surface so as to communicate with the internal space 11; a flap portion 30 which is formed to have a plate shape; and a flap radial-expansion prevention portion 40 of which one end portion (the first end) 41 is connected to an extension portion 31 of the flap portion 30. The flap portion 30 has the extension portion 31 which extends radially outward from the side surface of the main body 10 so as to engage with tissue and a fixed portion 32 which is fixed to the main body 10 while being continuous with the extension portion 31.

The main body 10 is formed to have a tubular shape. A distal end opening 14 which is formed at a distal end surface 21 of the main body 10 and a proximal end opening 15 which is formed at a proximal end surface 22 of the main body 10 communicate with the internal space 11. A tubular path of the main body 10 is configured to include the above-described internal space 11, the distal end opening 14, and the proximal end opening 15.

The side surface of a distal end portion of the main body 10 is provided with a second side opening 16 which communicates with the internal space 11. The side surface of the distal end portion of the main body 10 is formed so as to decrease in diameter towards the distal end side.

The main body 10 is formed from a resin material such as urethane and polyethylene having elasticity, flexibility, and biocompatibility.

The extension portion 31 extends from the fixed portion 32 to the distal end side in its natural state, and the flap portion 30 extends radially outward from the side surface of the main body 10, thereby being open. In other words, the flap portion 30 is formed so as to be in an open state in its natural state. The aforementioned term "its natural state" refers to a state where no external force other than gravity and a load caused by the flap radial-expansion prevention portion 40 is applied to the flap portion 30.

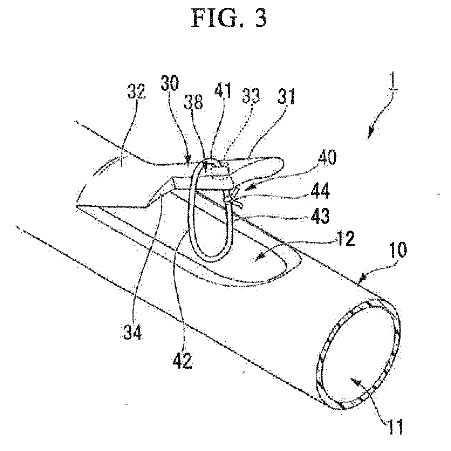

As illustrated in FIGS. 2 and 3, a penetration hole (the engagement portion) 33 extending in the thickness direction of the flap portion 30 is formed in the extension portion 31 of the flap portion 30. A step portion 34 is formed in the flap portion 30 by causing the width on the extension portion 31 side to be narrower than the width on the fixed portion 32 side. The length of the flap portion 30 in the width direction in the step portion 34 is greater than the outer diameter of a filamentous member 43 described below. In the present embodiment, one flap portion 30 is provided on a proximal end side of the main body 10.

The flap portion 30 and the side opening 12 are formed by cutting and raising the side surface of the main body 10. In other words, a tongue-shaped portion (not illustrated) is formed by making a notch on the side surface of the main body 10, thereby forming the above-described step portion 34 in the tongue-shaped portion. When the tongue-shaped portion is subjected to known heat processing or the like, the tongue-shaped portion has a bending tendency of opening radially outward in its natural state, thereby forming the flap portion 30. Therefore, the side opening 12 is formed on the flap portion 30 side with respect to the longitudinal axis C. When the flap portion 30 is pressed radially from the outside toward the longitudinal axis C, the flap portion 30 is elastically transformed (moved) to the longitudinal axis C side so as to be in a closed state extending along the longitudinal axis C, and thus, the flap portion 30 is accommodated in the side opening 12.

In this manner, the flap portion 30 is transformable between the open state and the closed state.

In the present embodiment, as illustrated in FIG. 1, one flap portion 50 is provided on the distal end side of the main body 10.

The flap portion 50 is formed to have a plate shape, and has an extension portion 51 which extends radially outward from the side surface of the main body 10 and a fixed portion 52 which is fixed to the main body 10 while being continuous with the extension portion 51. The fixed portion 52 of the flap portion 50 is fixed to the side surface of the main body 10 being closer to the distal end side than the second side opening 16. The extension portion 51 extends from the fixed portion 52 to the proximal end side in its natural state, and the flap portion 50 extends radially outward from the side surface of the main body 10, thereby being open. In other words, the flap portion 50 is formed so as to be in the open state in its natural state.

Similar to the flap portion 30 and the side opening 12, the flap portion 50 and the second side opening 16 are formed by making notches on the side surface of the main body 10. When the flap portion 50 is pressed radially from the outside toward the longitudinal axis C of the main body 10, the flap portion 50 is elastically transformed to the longitudinal axis C side, and thus, the flap portion 50 is accommodated in the second side opening 16.

The flap portions 30 and 50 are formed from the same material as that of the main body 10.

As illustrated in FIGS. 2 and 3, the flap radial-expansion prevention portion 40 has a configuration in which the filamentous member 43 is annularly formed. More specifically, the flap radial-expansion prevention portion 40 has a configuration in which the filamentous member 43 is bent, a folded-back portion is formed in the one end portion 41, end portions of the filamentous member 43 on both sides with respect to the one end portion 41 are tied, and a knot portion 44 is formed. The filamentous member 43 can be formed from a resin of nylon, polypropylene, polydioxanone, and the like having flexibility and biocompatibility.

The one end portion 41 of the filamentous member 43 is inserted through the penetration hole 33 of the flap portion 30, and the other end portion (second end portion) 42 of the filamentous member 43 is disposed closer to the extension portion 31 side than the step portion 34 side in the flap portion 30. The flap radial-expansion prevention portion 40 is attached to the flap portion 30 by forming the knot portion 44 in a state where one side of the filamentous member 43 is inserted through the penetration hole 33.

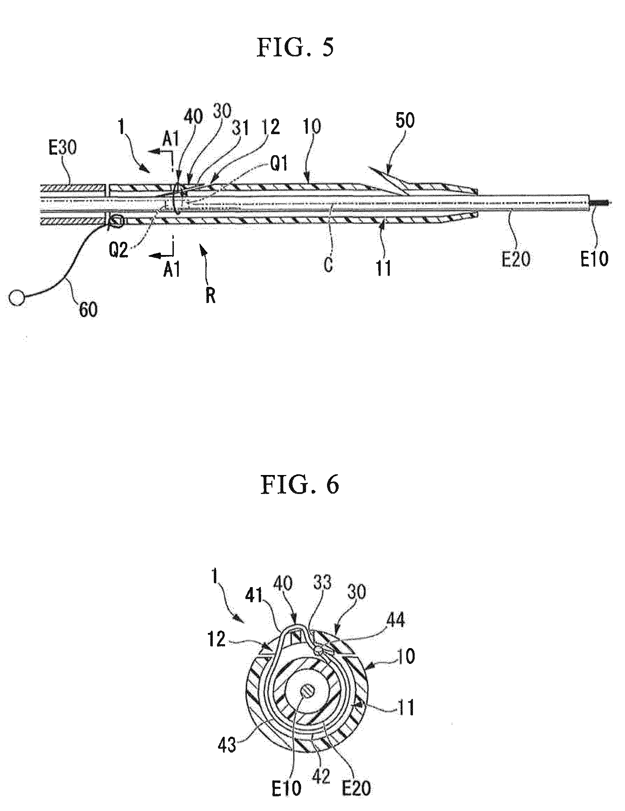

As illustrated in FIG. 2, when the flap portion 30 is in the open state, the flap radial-expansion prevention portion 40 in its entirety is arranged outside the internal space 11 of the main body 10. As illustrated in FIGS. 5 and 6, in the flap radial-expansion prevention portion 40, when the flap portion 30 is transformed to the longitudinal axis C side so as to be in the closed state, the other end portion 42 which is continuous with the one end portion 41 passes through the side opening 12 on the internal space 11 side.

As illustrated in FIGS. 2 and 3, the extension portion 31 of the flap portion 30 is provided with a pressed portion 38 which is pressed by the filamentous member 43 so that a force for transforming the flap portion 30 from the open state to the closed state is applied to the flap portion 30. The above-described penetration hole 33 is formed in the pressed portion 38 of the flap portion 30 and has a configuration in which the pressed portion 38 is prevented from being positionally misaligned in the flap portion 30 when the filamentous member 43 is inserted through the penetration hole 33.

In the present embodiment, as illustrated in FIG. 1, an end portion of a grip portion 60 is attached to a proximal end portion of the main body 10. The grip portion 60 can be formed from the same material as that of the filamentous member 43.

In a direction along the longitudinal axis C of the main body 10, radiopaque markers may be embedded in correspondent positions to the extension portion 31 of the flap portion 30 and the extension portion 51 of the flap portion 50 of the main body 10.

A description will be given below regarding an operation of the stent 1 having the above-described configuration, with reference to an example of a technique of placing the stent 1 in the bile duct.

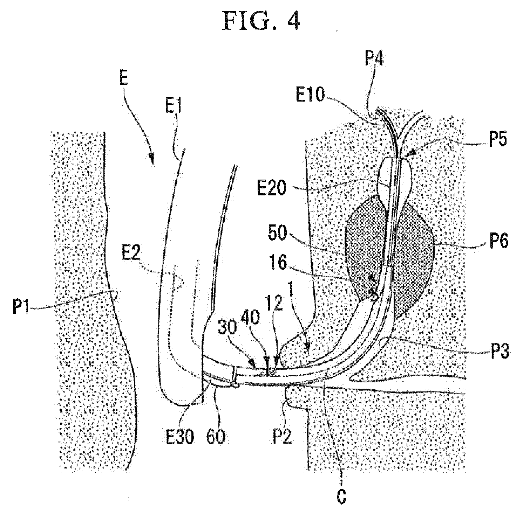

First, a user such as an operator inserts a side-view type endoscope into a body cavity of a patient through a natural opening such as the mouth of the patient, and as illustrated in FIG. 4, a distal end portion of an insertion portion E1 of an endoscope E is caused to move forward to the vicinity of a duodenal papilla P2 through a duodenum P1.

The user then inserts a guide wire E10 into a channel E2 through a forceps opening (not illustrated) of the endoscope E, and causes the distal end portion of the guide wire E10 to protrude toward the duodenal papilla P2 from the distal end opening of the channel E2 while suitably operating a raising base (not illustrated). Thereafter, the distal end portion of the guide wire E10 is inserted into a hepatic duct P4 through the inside of a bile duct P3 from the duodenal papilla P2. Generally, the inner diameter of the hepatic duct P4 is smaller than the inner diameter of the bile duct P3.

In the proximal end portion of the guide wire E10, a guide sheath (the insertion member) E20 externally fits around the guide wire E10. The guide sheath E20 is pushed therein (is moved to the distal end side) while being in a state where the positions of the insertion portion E1 and the guide wire E10 are maintained. When the distal end portion of the guide sheath E20 abuts on a connection site P5 between the hepatic duct P4 and the bile duct P3, the guide sheath E20 can be pushed no further than the abutted site.

The user checks a shape of a stenosed site P6 of the bile duct P3 through radioscopy and selects the stent 1 of which the length from the extension portion 31 of the flap portion 30 to the extension portion 51 of the flap portion 50 is substantially the same as or greater than the length of the stenosed site P6 along the bile duct P3 when each of the flap portions 30 and 50 is opened.

In the proximal end portion of the guide sheath E20, the stent 1 externally fits around the guide sheath E20 from the distal end portion side. In other words, the guide sheath E20 is inserted into the internal space 11 of the main body 10 of the stent 1. The guide sheath E20 is movable along the longitudinal axis C in the internal space 11 of the main body 10.

In this case, as illustrated in FIGS. 5 and 6, the user causes the extension portion 31 of the flap portion 30 to elastically transform to the longitudinal axis C side by pressing the flap portion 30 with one's hand or the like radially from the outside toward the longitudinal axis C of the main body 10, and the flap portion 30 is accommodated in the side opening 12. As a result thereof, the other end portion 42 of the flap radial-expansion prevention portion 40 passes through the side opening 12 and is arranged inside the internal space 11. In this case, the guide sheath E20 which is inserted into the internal space 11 of the main body 10 can be inserted through the annular filamentous member 43 which is arranged inside the internal space 11. The expression "the guide sheath E20 can be inserted through the annular filamentous member 43" refers to a state in which the length of a portion in the filamentous member 43 which is arranged inside the internal space 11 becomes equal to or greater than the length of the outer circumference of the guide sheath E20.

When the stent 1 is pushed against the guide sheath E20 by using a pusher catheter E30 or the like as described below, the guide sheath E20 is inserted through the internal space 11 of the main body 10 and is also inserted through the annular flap radial-expansion prevention portion 40. The guide sheath E20 is inserted through the flap radial-expansion prevention portion 40 when the guide sheath E20 which is inserted through the internal space 11 of the main body 10 is arranged within a reference range R in the direction along the longitudinal axis C, as illustrated in FIG. 5. The aforementioned term "the reference range R" refers to a range from a state where the guide sheath E20 is disposed at a position Q1 with respect to the stent 1, that is, the distal end portion of the guide sheath E20 coincides with the flap radial-expansion prevention portion 40, to a state where the guide sheath E20 is disposed at a position Q2 with respect to the stent 1, that is, the proximal end portion of the guide sheath E20 coincides with the flap radial-expansion prevention portion 40.

When the guide sheath E20 is arranged within the reference range R with respect to the stent 1, the guide sheath E20 is inserted through the annular flap radial-expansion prevention portion 40 so that the other end portion 42 of the flap radial-expansion prevention portion 40 engages with (is held on) the longitudinal axis C side, and the guide sheath E20 performs maintaining in a state where the extension portion 31 of the flap portion 30 elastically transforms to the longitudinal axis C side. In this case, the flap portion 30 is accommodated in the side opening 12, and the closed state of the flap portion 30 is maintained.

Meanwhile, when the guide sheath E20 which is inserted through the internal space 11 of the main body 10 is arranged outside of the reference range R in the direction along the longitudinal axis C, engagement between the flap radial-expansion prevention portion 40 and the guide sheath E20 is cancelled. In this case, the extension portion 31 of the flap portion 30 moves in a direction away from the longitudinal axis C due to its elastic force, thereby being opened. The expression "when the guide sheath E20 is arranged outside of the reference range R in the direction along the longitudinal axis C" specifically refers to a state where the guide sheath E20 is disposed closer to the proximal end side than the position Q1 with respect to the stent 1, and a state where the guide sheath E20 is disposed closer to the distal end side than the position Q2 with respect to the stent 1.

In this state, the guide sheath E20 cannot be inserted through the flap radial-expansion prevention portion 40, and the guide sheath E20 cannot engage with the flap radial-expansion prevention portion 40.

The distal end surface of the pusher catheter E30 abuts on the proximal end surface 22 of the stent 1, and the pusher catheter E30 externally fits around the guide sheath E20. The pusher catheter E30 is a general catheter having a tubular shape, and the pusher catheter E30 having an outer diameter substantially equal to the outer diameter of the main body 10 of the stent 1.

The pusher catheter E30 is pushed against the insertion portion E1 and the guide sheath E20, and the stent 1 is moved to the distal end side in the channel E2. In this case, the guide sheath E20 is arranged within the reference range R without causing the guide sheath E20 to move closer to the proximal end side than the position Q1 with respect to the stent 1. Therefore, the guide sheath E20 engages with the flap radial-expansion prevention portion 40, and the closed state of the flap portion 30, that is, a state where the flap portion 30 is accommodated in the side opening 12 is maintained.

As illustrated in FIG. 4, an inner wall of the bile duct P3 presses the flap portion 50, which transforms to the longitudinal axis C side when the stent 1 is introduced into the bile duct P3 since the flap portion 50 on the distal end side is formed so as to extend to the proximal end side and to be open radially outward. In this manner, since the flap portion 50 transforms to the longitudinal axis C side along the inner wall of the bile duct P3 and the flap portion 30 is in the closed state, it is possible to easily introduce the stent 1 in its entirety into the bile duct P3 while requiring no special treatment device for covering an outer circumferential surface of the stent 1 when introducing the stent 1.

When a flap 50 of the stent 1 reaches the stenosed site P6, the flap portion 50 is pressed by the inner wall of the stenosed site P6, transforms to the longitudinal axis C side further, and is accommodated in the second side opening 16, thereby being in the closed state.

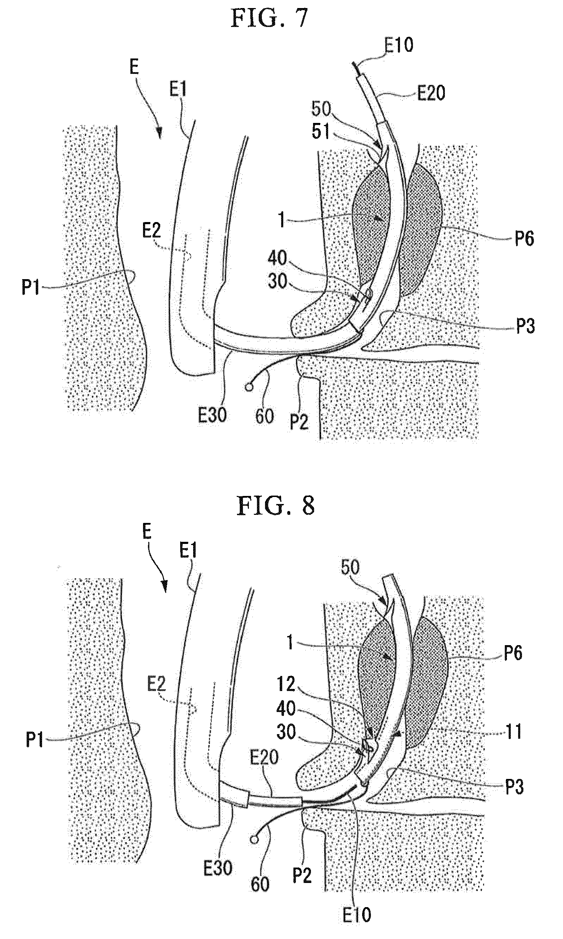

The user introduces the stent 1 while checking the positions of the flap portions 30 and 50 through radioscopy with reference to the aforementioned markers and the like, and the user disposes the stent 1 at a position where the flap portion 50 passes by the stenosed site P6, as illustrated in FIG. 7. The pressing applied by the inner wall of the stenosed site P6 is cancelled, and the flap portion 50 is in the open state, thereby engaging with a back side of the stenosed site P6. In this case, the proximal end portion of the grip portion 60 protrudes into a lumen of the duodenum P1 from the duodenal papilla P2.

In a state where positions of the insertion portion E1 of the endoscope E and the pusher catheter E30 are maintained, the guide wire E10 returns to the proximal end side and is removed from the forceps opening, as illustrated in FIG. 8. In a state where a position of the pusher catheter E30 is maintained, the guide sheath E20 returns to the proximal end side. Since the distal end surface of the pusher catheter E30 abuts on the proximal end surface 22 of the stent 1, the stent 1 does not move to the proximal end side. The guide sheath E20 is arranged outside of the reference range R at the time point when the guide sheath E20 moves closer to the proximal end side than the aforementioned position Q1 with respect to the stent 1. In this case, engagement between the flap radial-expansion prevention portion 40 and the guide sheath E20 is cancelled, and the flap portion 30 is in the open state.

Whether or not the flap portion 30 is in a completely open state depends on a state of the inner wall of the bile duct P3 around the flap portion 30.

When the flap portion 30 is in the open state, the flap radial-expansion prevention portion 40 passes through the side opening 12 and is drawn out of the internal space 11. Since the stent 1 of which the length from the extension portion 31 of the flap portion 30 to the extension portion 51 of the flap portion 50 is set as described above has been selected, the flap portion 30 also engages with a front side of the stenosed site P6.

The user then causes the guide sheath E20 and the pusher catheter E30 to pull back to the proximal end side and takes out the guide sheath E20 and the pusher catheter E30 through the forceps opening of the endoscope E. The user takes out the insertion portion E1 of the endoscope E from the inside of a body cavity of the patient and performs appropriate treatment, thereby ending the technique of placing the stent 1.

While placing the stent 1, bile produced in the liver flows inside the tubular path in the main body 10 of the stent 1, in a portion corresponding to the stenosed site P6 in the bile duct P3 and is supplied to the lumen of the duodenum P1.

The procedure at the time of collecting the placing stent 1 is as follows. As described above, the distal end portion of the insertion portion E1 of the endoscope E is caused to move forward to the vicinity of the duodenal papilla P2 through the duodenum P1. Grip forceps which is not illustrated or the like are inserted through the channel E2 via the forceps opening. The proximal end portion of the grip portion 60 is gripped with the grip forceps, and the grip forceps are caused to pull back to the insertion portion E1, thereby pulling out the stent 1 from the inside of the bile duct P3. The grip forceps and the insertion portion E1 are removed from the inside of the body cavity of the patient.

According to the stent 1 of the present embodiment, when the guide sheath E20 inserted through the internal space 11 of the main body 10 is arranged within the reference range R in the direction along the longitudinal axis C, the guide sheath E20 engages with the flap radial-expansion prevention portion 40, thereby maintaining the closed state in which the extension portion 31 of the flap portion 30 has transformed to the longitudinal axis C side. When the guide sheath E20 is arranged within the reference range R, the closed state of the flap portion 30 is maintained. Therefore, it is possible to adjust the positions of the guide sheath E20 and the stent 1 in the direction along the longitudinal axis C within the reference range R while maintaining the closed state of the flap portion 30.

When the stent 1 is disposed at a desired position, the stent 1 is held by the pusher catheter E30 so as not to move to the proximal end side, and the guide sheath E20 is pulled back to the proximal end side. When the guide sheath E20 is arranged outside of the reference range R with respect to the stent 1, engagement between the flap radial-expansion prevention portion 40 and the guide sheath E20 is cancelled, and the flap portion 30 is in the closed state, thereby engaging with the stenosed site P6 on the front side.

In this manner, since the closed state of the flap portion 30 can be maintained when introducing the stent 1 into the bile duct P3, it is possible to prevent the flap portion 30 from engaging with the duodenal papilla P2 or the inner wall of the bile duct P3 when introducing the stent 1. As a result of this, it is possible to enhance introduction characteristics when introducing the stent 1 into the bile duct P3.

Since no special treatment device for covering the outer circumferential surface of the stent 1 is required, it is possible to achieve a compact configuration in its entirety.

Since placement of the stent 1 can be performed by using the tubular-shaped pusher catheter E30, placement of the stent 1 can be easily performed.

Since the flap radial-expansion prevention portion 40 has a configuration in which the filamentous member 43 is annularly formed, the flap radial-expansion prevention portion 40 can be easily configured, and thus, the manufacturing cost of the stent 1 can be reduced.

When the flap portion 30 is in the open state, the flap radial-expansion prevention portion 40 is arranged outside the internal space 11 of the main body 10. Therefore, when bile flows in the internal space 11 of the stent 1, it is possible to prevent the flap radial-expansion prevention portion 40 from hindering the flow of the bile.

Since the penetration hole 33 is formed in the extension portion 31 of the flap portion 30, the pressed portion 38 of the flap portion 30 which is pressed by the filamentous member 43 can be prevented from being positionally misaligned when transforming the flap portion 30 from the open state to the closed state.

When the filamentous member 43 is inserted through the penetration hole 33 of the flap portion 30, the flap radial-expansion prevention portion 40 can be prevented from deviating from the flap portion 30, and the flap radial-expansion prevention portion 40 can be reliably attached to the flap portion 30.

Since the flap portion 30 and the side opening 12 are formed by cutting and raising the side surface of the main body 10, the flap portion 30 and the side opening 12 can be easily formed at one time from the main body 10 which is formed to have a tubular shape.

The filamentous member 43 is disposed closer to the extension portion 31 side than the step portion 34 side of the flap portion 30. Therefore, when the flap portion 30 transforms to the open state or the closed state, the flap portion 30 can be prevented from coming into being difficult to transform from contacting with the main body 10 of the filamentous member 43.

The configuration of the stent 1 of the present embodiment can be variously modified as described below.

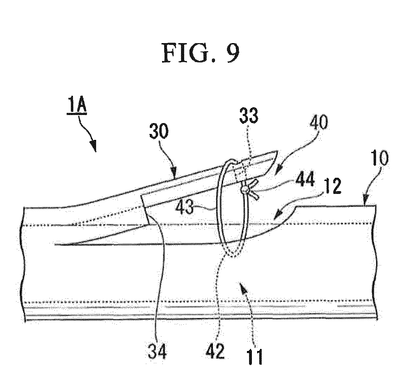

For example, as in a stent 1A illustrated in FIG. 9, since bile can flow in the internal space 11 when the stent 1A has placed, when the flap portion 30 is in the open state, the other end portion 42 of the flap radial-expansion prevention portion 40 may be arranged in the internal space 11 of the main body 10. In accordance with such a configuration, bile can flow in the internal space 11 when the stent 1A has placed.

According to the stent 1A having such a configuration, it is possible to cause introduction characteristics to be favorable while requiring no special treatment device at the time of introduction.

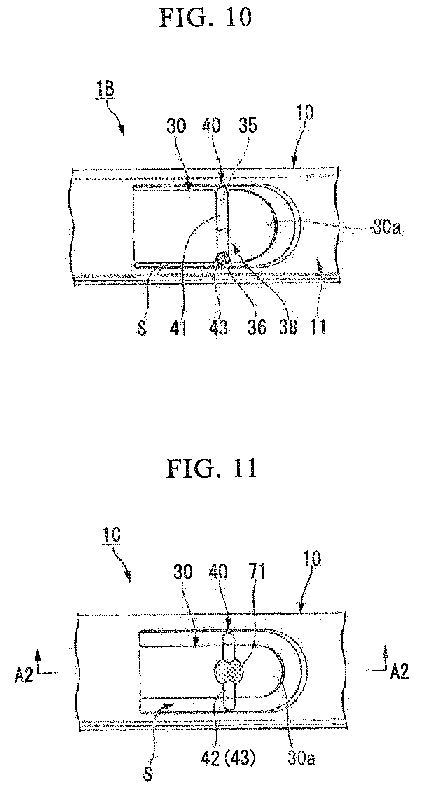

As in a stent 1B illustrated in FIG. 10, groove portions (engagement portions) 35 and 36 may be formed in the pressed portion 38 of the flap portion 30 in place of the penetration hole 33 and the step portion 34 so as to have a configuration in which the filamentous member 43 of the flap radial-expansion prevention portion 40 is arranged inside the groove portions 35 and 36. The groove portions 35 and 36 are respectively formed on the side surfaces at the ends of the flap portion 30 in the width direction so as to penetrate the flap portion 30 in the thickness direction. The sizes of the groove portions 35 and 36 when viewed in the thickness direction illustrated in FIG. 10 are formed to be substantially equal to that of a cross-section orthogonal to the filamentous member 43 in the longitudinal direction.

The one end portion 41 of the flap radial-expansion prevention portion 40 is arranged on a surface 30a of the main body 10 on a side opposite to the internal space 11 in the flap portion 30. The filamentous member 43 may be configured to be fixed to the groove portions 35 and 36 by press-fitting the filamentous member 43 into the groove portions 35 and 36.

According to the stent 1B having such a configuration, it is possible to reliably prevent the filamentous member 43 and the pressed portion 38 from moving to the distal end side or the proximal end side with respect to the flap portion 30. Since there is no need to form the knot portion 44 after the filamentous member 43 is inserted through the penetration hole 33 of the flap portion 30 so that the filamentous member 43 can be attached to the flap portion 30 after the knot portion 44 is formed therein in advance, it is possible to easily form the knot portion 44 in the filamentous member 43.

It is preferable that the width of a gap S formed between the flap portion 30 and the main body 10 on the width direction side of the flap portion 30 be sufficiently smaller than the outer diameter of the filamentous member 43. According to the aforementioned configuration, even when the filamentous member 43 deviates from the groove portions 35 and 36, the filamentous member 43 is likely to move to the distal end side or the proximal end side through the gap S.

In the present modification example, the groove portions 35 and 36 are respectively formed at the ends of the flap portion 30. However, a configuration is acceptable as long as at least the groove portion 35 is formed in the flap portion 30. This is because an effect similar to that described above can be achieved by arranging the filamentous member 43 in only the groove portion 35.

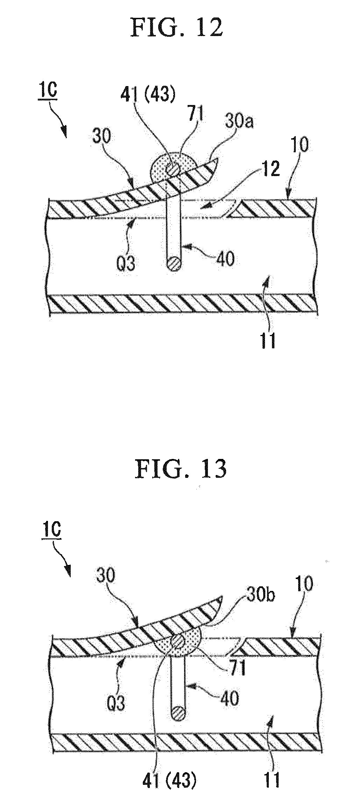

As in a stent 1C illustrated in FIGS. 11 and 12, the width of the gap S may be greater than the outer diameter of the filamentous member 43, and the one end portion 41 of the flap radial-expansion prevention portion 40 arranged on the surface 30a of the flap portion 30 may be fixed to the flap portion 30 by using a fixed portion 71 such as an adhesive. A position Q3 indicated by the two-dot chain line in FIG. 12 implies a position when the flap portion 30 is in the closed state in which the flap portion 30 is accommodated in the side opening 12.

In this modification example, the penetration hole 33, the step portion 34, and the groove portions 35 and 36 described above are not formed in the flap portion 30. It is preferable that the width of the gap S be greater than the outer diameter of the filamentous member 43 so as to prevent the filamentous member 43 from being interfered with by the main body 10 when the flap portion 30 transforms to the open state or the closed state.

According to the stent 1C having such a configuration, the flap radial-expansion prevention portion 40 can be attached to the flap portion 30 without forming the penetration hole 33 and the like in the flap portion 30.

In the present modification example, as illustrated in FIG. 13, the one end portion 41 of the flap radial-expansion prevention portion 40 arranged on a surface 30b on the internal space 11 side of the main body 10 in the flap portion 30 may be fixed to the flap portion 30 by using the fixed portion 71. In this example, the flap radial-expansion prevention portion 40 in its entirety is disposed closer to the internal space 11 side than the flap portion 30.

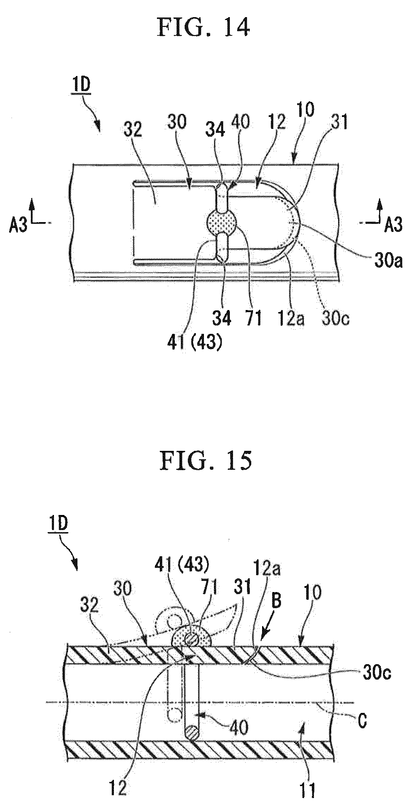

As in a stent 1D illustrated in FIGS. 14 and 15, the step portions 34 may be formed on both sides of the flap portion 30 in the width direction, and the one end portion 41 of the flap radial-expansion prevention portion 40 arranged on the surface 30a of the flap portion 30 may be fixed to the flap portion 30 by using the fixed portion 71. FIGS. 14 and 15 illustrate the closed state of the flap portion 30 in which the flap portion 30 is accommodated in the side opening 12. FIG. 15 illustrates the shape of the flap portion 30 in the open state indicated by the two-dot chain line.

A side surface 12a forming an edge portion of the side opening 12 on the distal end side in the main body 10 is inclined so as to be close to the longitudinal axis C toward the proximal end side. A side surface 30c forming an edge portion of the flap portion 30 on the distal end side when being accommodated in the side opening 12 is inclined so as to be close to the longitudinal axis C toward the proximal end side. In other words, when the flap portion 30 is accommodated in the side opening 12, the side surface 12a of the main body 10 and the side surface 30c of the flap portion 30 face each other. In this case, the distance between the side surface 12a and the side surface 30c is smaller than the outer diameter of the filamentous member 43.

When the flap portion 30 is cut and raised from the main body 10 as described above, the flap portion 30 is separated from the main body 10 by making a cut obliquely to the longitudinal axis C as indicated by the arrow B in FIG. 15. In this manner, when the flap portion 30 is accommodated in the side opening 12, the side surfaces 12a and 30c become face-to-face surfaces which are formed obliquely to the longitudinal axis C. The side surface 12a and the side surface 30c overlap each other when the flap portion 30 illustrated in FIG. 14 is radially viewed in a planar manner.

The filamentous member 43 on each side with respect to the one end portion 41 is disposed closer to the extension portion 31 side than the step portion 34 side in the flap portion 30.

According to the stent 1D having such a configuration, when the side surfaces 12a and 30c overlap each other when being radially viewed while being in the closed state in which the flap portion 30 is accommodated in the side opening 12, it is possible to prevent the filamentous member 43 from coming off from the distal end side of the flap portion 30.

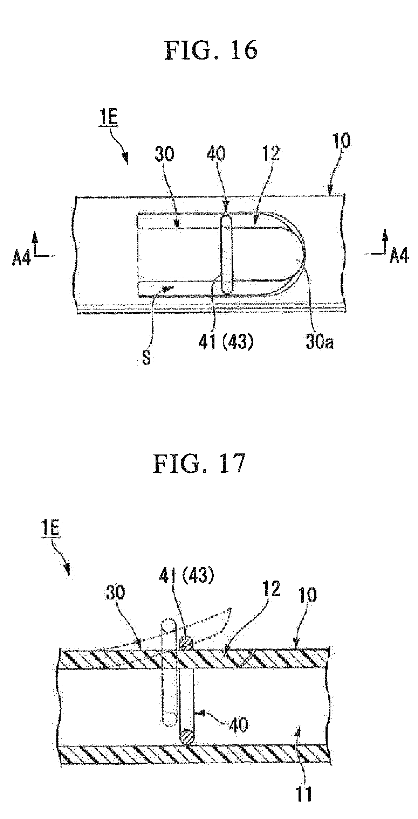

As in a stent 1E illustrated in FIGS. 16 and 17, the one end portion 41 of the filamentous member 43 may be arranged on the surface 30a of the flap portion 30. FIGS. 16 and 17 illustrate the closed state in which the flap portion 30 is accommodated in the side opening 12. FIG. 17 illustrates the shape of the flap portion 30 in the open state indicated by the two-dot chain line.

The width of the gap S is slightly smaller than the outer diameter of the filamentous member 43. When the filamentous member 43 is inserted through the gap S, the filamentous member 43 cannot move to the distal end side or the proximal end side with respect to the flap portion 30 and the main body 10 due to a frictional force applied between the flap portion 30 and the main body 10, and the filamentous member 43.

### (Second Embodiment)

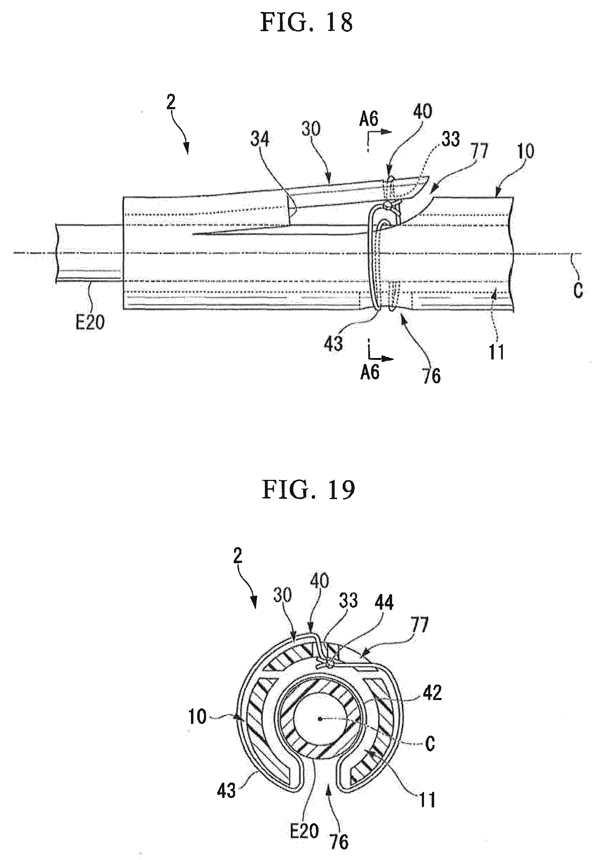

A second embodiment of the present invention will be described with reference to FIGS. 18 to 21. The same reference numerals and signs will be applied to the same portions as in the above-described embodiment, and the description thereof will be omitted. A description will be given regarding only the differences therebetween.

As illustrated in FIGS. 18 and 19, in a stent 2 of the present embodiment, a side opening 76 and a communication opening 77 are formed in the main body 10 in place of the side opening 12 which is formed in the main body 10 of the stent 1 in the first embodiment. The side opening 76 is formed on a side opposite to the flap portion 30 with respect to the longitudinal axis C. The communication opening 77 is formed at a position facing the flap portion 30 of the main body 10 so as to communicate with the internal space 11.

In the present embodiment, the filamentous member 43 of the flap radial-expansion prevention portion 40 is longer than that in the first embodiment. The filamentous member 43 inserted through the penetration hole 33 of the flap portion 30 is wrapped around the main body 10 in a crawling manner along the outer circumferential surface while being in a state where the guide sheath E20 is not inserted.

The stent 2 having such a configuration is adopted in the technique as follows.

The flap portion 30 transforms to the longitudinal axis C side so as to be in the closed state. The other end portion 42 of the filamentous member 43 which is wrapped around the main body 10 along the outer circumferential surface passes through the side opening 76 and is disposed on the internal space 11 side. The filamentous member 43 is disposed on the inner surface of the internal space 11 along the circumferential direction.

The guide sheath E20 is inserted into the internal space 11 of the main body 10 of the stent 2. When the guide sheath E20 is disposed on the side opening 76 side of the filamentous member 43 which is arranged on the inner surface of the internal space 11 along the circumferential direction, the other end portion 42 of the filamentous member 43 engages with (is held by) the guide sheath E20 so as not to move to the side opening 76 side.

Since the filamentous member 43 arranged in the internal space 11 is configured not to move to the side opening 76 side, the flap radial-expansion prevention portion 40 engages with the guide sheath E20, and the closed state of the flap portion 30 is maintained.

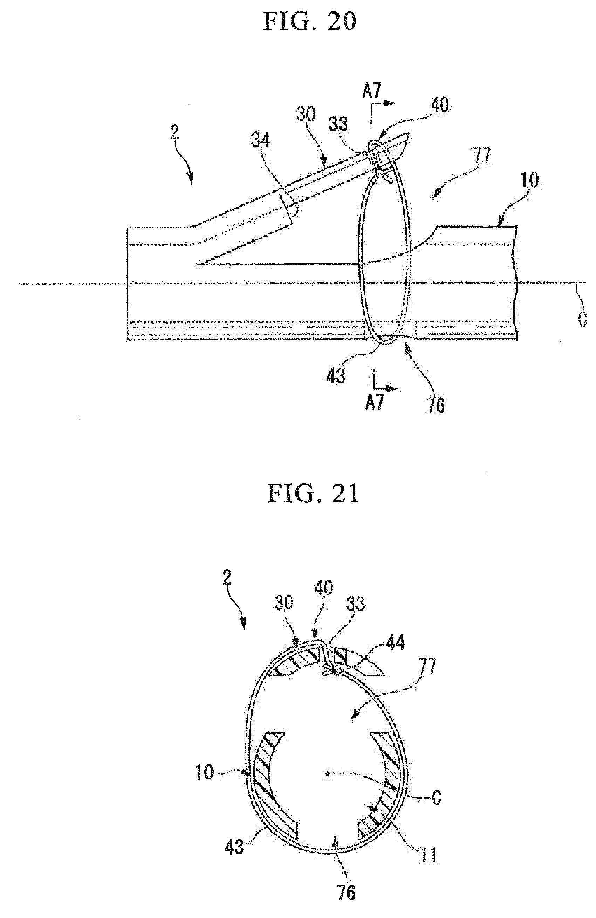

The guide sheath E20 is pulled back to the proximal end side with respect to the stent 2 by using the pusher catheter E30 which is not illustrated. As illustrated in FIGS. 20 and 21, engagement between the flap radial-expansion prevention portion 40 and the guide sheath E20 is cancelled, and the flap portion 30 is in the open state due to its elastic force. The filamentous member 43 arranged in the internal space 11 passes through the side opening 76 and is drawn out of the main body 10.

As described above, according to the stent 2 of the present embodiment, it is possible to enhance introduction characteristics by maintaining the closed state of the flap portion 30 using the guide sheath E20 while requiring no special treatment device at the time of introduction into the bile duct P3.

The filamentous member 43 is arranged outside the internal space 11 when the guide sheath E20 is pulled out and the flap portion 30 is in the open state. Therefore, the flap radial-expansion prevention portion 40 is prevented from hindering the flow of the bile when bile flows in the internal space 11 so that the stent 2 can be unlikely to be clogged.

### (Third Embodiment)

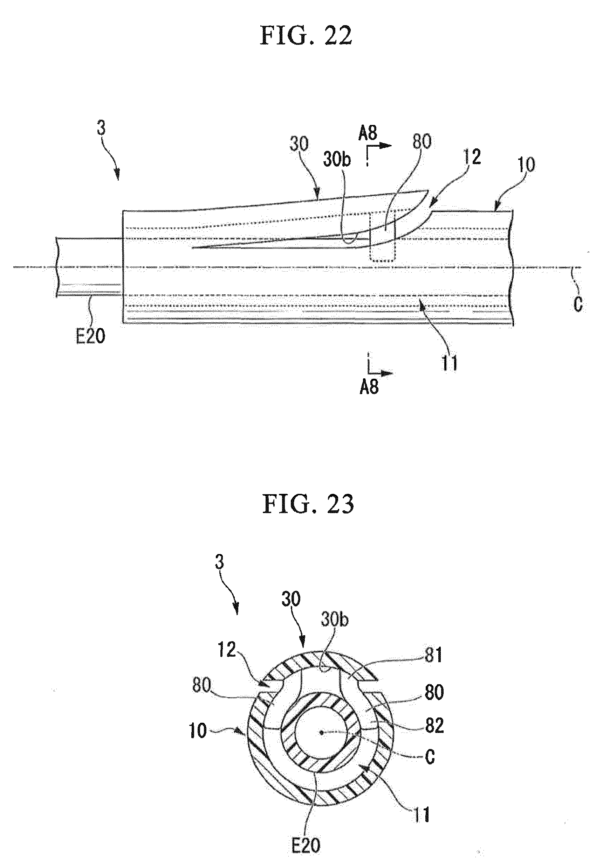

A third embodiment of the present invention will be described with reference to FIGS. 22 to 25. The same reference numerals and signs will be applied to the same portions as in the above-described embodiment, and the description thereof will be omitted. A description will be given regarding only the differences therebetween.

As illustrated in FIGS. 22 and 23, a stent 3 of the present embodiment is provided with a flap radial-expansion prevention portion 80 in place of the flap radial-expansion prevention portion 40 of the stent 1 in the first embodiment. In this example, the penetration hole 33 and the step portion 34 are not formed in the flap portion 30.

In the present embodiment, a pair of the flap radial-expansion prevention portions 80 formed to have belt shapes are provided. The flap radial-expansion prevention portions 80 can be formed from the same material as that of the flap portion 30. The pair of flap radial-expansion prevention portions 80 are disposed side-by-side interposing the longitudinal axis C therebetween in the width direction of the flap portion 30. One end portion 81 of each flap radial-expansion prevention portion 80 is connected to the surface 30b on the internal space 11 side in the flap portion 30 by using a known adhesive or the like.

The distance between the pair of the flap radial-expansion prevention portions 80 in the width direction is smaller than the outer diameter of the guide sheath E20. The lengths of the pair of the flap radial-expansion prevention portions 80 in their entirety in the width direction are smaller than the length of the side opening 12 in the width direction.

The stent 3 having such a configuration is adopted in the technique as follows.

The flap portion 30 transforms to the longitudinal axis C side so as to be in the closed state. In this case, each of the other end portions 82 of the flap radial-expansion prevention portions 80 passes through the side opening 12 and is arranged in the internal space 11.

The guide sheath E20 is inserted into the internal space 11 of the main body 10 of the stent 3. When the guide sheath E20 is inserted between the pair of the flap radial-expansion prevention portions 80, the other end portions 82 of the flap radial-expansion prevention portions 80 are individually opened so as to be separated from each other, and a frictional force is applied between the flap radial-expansion prevention portions 80 and the guide sheath E20. Each of the flap radial-expansion prevention portions 80 cannot move to the side opening 12 side with respect to the guide sheath E20 due to the frictional force thereof. Therefore, each of the flap radial-expansion prevention portions 80 engages with the guide sheath E20, and the closed state of the flap portion 30 is maintained.

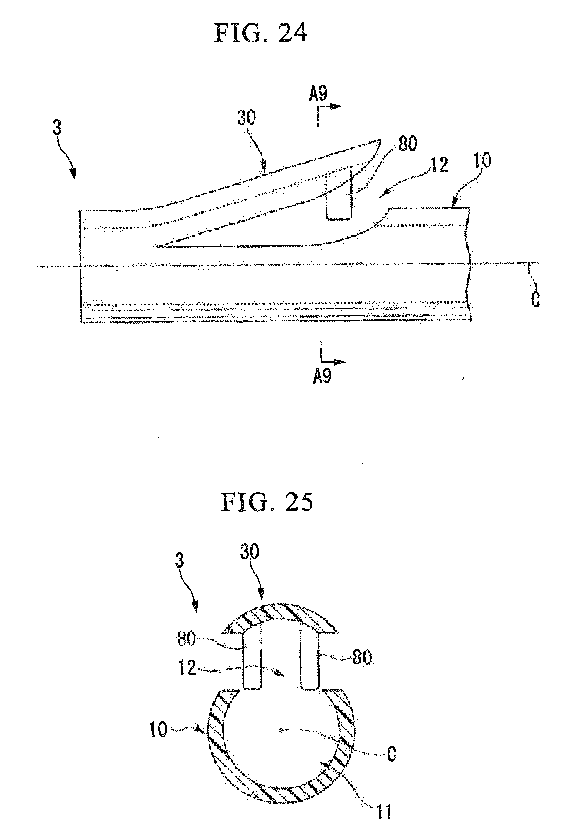

The guide sheath E20 is pulled back to the proximal end side with respect to the stent 3 by using the pusher catheter E30 which is not illustrated. As illustrated in FIGS. 24 and 25, engagement between the flap radial-expansion prevention portions 80 and the guide sheath E20 is cancelled, and the flap portion 30 is in the open state due to its elastic force. Each of the flap radial-expansion prevention portions 80 arranged in the internal space 11 passes through the side opening 12 and moves to the outer side of the main body 10.

As described above, according to the stent 3 of the present embodiment, it is possible to enhance introduction characteristics by maintaining the closed state of the flap portion 30 using the guide sheath E20 while requiring no special treatment device at the time of introduction into the bile duct P3.

The present embodiment may have a configuration in which a frictional force is applied between the flap radial-expansion prevention portions 80 and the main body 10 as well when the guide sheath E20 is inserted between the pair of the flap radial-expansion prevention portions 80.

Hereinbefore, descriptions are given in detail from the first embodiment to the third embodiment of the present invention with reference to the drawings. However, the specific configurations are not limited to the embodiments. The embodiments include changes, combinations, deletions, and the like to the configurations without departing from the gist and the scope of the present invention. Moreover, it is needless to mention that each of the configurations presented in the embodiments can be suitably combined and utilized.

For example, in the first embodiment and the second embodiment, the flap portion 30 is accommodated in the side opening 12 when the guide sheath E20 engages with the flap radial-expansion prevention portion 40. However, a configuration is acceptable as long as the extension portion 31 of the flap portion 30 moves to the longitudinal axis C side when the guide sheath E20 engages with the flap radial-expansion prevention portion 40.

In the above-described configuration, one flap portion 30 is provided on the proximal end side of the main body 10, and one flap portion 50 is provided on the distal end side of the main body 10. However, the number of the flap portions 30 and 50 to be provided in the main body 10 is not limited and may be two or more.

The guide sheath E20 is configured to be a linear member. However, the guide wire E10 may be a linear member, and the flap radial-expansion prevention portion 40 may be engaged and disengaged by using the guide wire E10.

In the above-described embodiment, the flap portion 30 is configured to be formed by cutting and raising the side surface of the main body 10. However, the main body may be formed to have a tubular shape, and a side opening portion may be formed on the side surface of the proximal end portion of the main body. The stent may be formed to have a configuration in which a fixed portion of a flap portion which is formed separately from the main body is fixed to the main body by performing glueing, heat welding, or the like.

The main body 10 may be provided with no flap portion 50. This is because the main body 10 can engage with a stenosed site by using an adhesive or the like with respect to a back side of the stenosed site. The stent may be provided with no grip portion 60.

The filamentous member may be formed from a material such as absorbable Dexon (registered trademark, manufactured by Covidien plc) which is decomposed by a body fluid or the like. Since the filamentous member is absorbed within a certain time period after placement is performed, it is possible for bile to be prevented from being difficult to flow in the internal space 11 even when the filamentous member is partially arranged in the internal space 11 at the time of placement.

In the above-described embodiment, descriptions are given regarding a case where the stent 1 is placed in the bile duct P3. However, the stent 1 may be used by being caused to place in the pancreatic duct.

The preferable embodiments of the present invention have been described above. However, the present invention is not limited to the embodiments and a modification example thereof. The configurations thereof can be subjected to addition, omission, replacement, and other changes without departing from the gist and the scope of the present invention.

The present invention is not limited to the above description and is only limited by the accompanying Claims.

### Industrial Applicability

According to the embodiments (including the modification example) described above, it is possible to maintain the closed state of a flap portion while requiring no special treatment device for covering an outer circumferential surface of a stent when introducing the stent into a human body.

### Reference Signs List

- 10: MAIN BODY (LONGITUDINAL MEMBER)
- 11: INTERNAL SPACE
- 12: SIDE OPENING
- 12a: SIDE SURFACE
- 30: FLAP PORTION
- 33: PENETRATION HOLE (ENGAGEMENT PORTION)
- 35,36: GROOVE PORTION (ENGAGEMENT PORTION)
- 38: PRESSED PORTION
- 40: FLAP RADIAL-EXPANSION PREVENTION PORTION

## Claims

1. A medical stent comprising:
a longitudinal member which has an internal space that extends along a longitudinal axis and a side opening that is formed on a side surface so as to communicate with the internal space, and allows an insertion member that is inserted into the internal space to move along the longitudinal axis;
a flap portion which has an extension portion that extends radially outward from the side surface of the longitudinal member so as to engage with tissue and a fixed portion which is fixed to the longitudinal member, being continuous with the extension portion, and is transformable between an open state in which the extension portion radially extends from the side surface of the longitudinal member and a closed state in which the extension portion extends along the longitudinal axis of the longitudinal member; and
a flap radial-expansion prevention portion which at least partially passes through the side opening and allows the insertion member to hold the flap portion in the internal space when the flap portion is in the closed state.

2. The medical stent according to Claim 1,
wherein the flap radial-expansion prevention portion is configured to be an annularly formed filamentous member,
wherein the side opening is formed on the flap portion side with respect to the longitudinal axis of the longitudinal member, and
wherein the flap radial-expansion prevention portion is held in the internal space by the insertion member when the insertion member is inserted through the annular flap radial-expansion prevention portion.

3. The medical stent according to Claim 1,
wherein the flap radial-expansion prevention portion is arranged outside the internal space of the longitudinal member when the flap portion is in the open state.

4. The medical stent according to Claim 1,
wherein the flap radial-expansion prevention portion is configured to be an annularly formed filamentous member,
wherein the side opening is formed on a side opposite to the flap portion with respect to the longitudinal axis of the longitudinal member, and
wherein the flap radial-expansion prevention portion is held in the internal space by the insertion member when the filamentous member passing through the side opening and being arranged along an inner surface of the internal space in a circumferential direction is held so as to prevent the insertion member from moving to the side opening side.

5. The medical stent according to Claim 1,
wherein the flap radial-expansion prevention portion is a filamentous member, and
wherein the flap portion is provided with a pressed portion which is pressed by the filamentous member so that a force for transforming the flap portion from the open state to the closed state is applied to the flap portion.

6. The medical stent according to Claim 5,
wherein the extension portion of the flap portion is provided with an engagement portion so as to prevent positional misalignment of the pressed portion in the flap portion.

7. The medical stent according to Claim 6,
wherein the engagement portion is a penetration hole which is formed in the pressed portion so as to extend in a thickness direction of the flap portion, and
wherein the filamentous member is inserted through the penetration hole.

8. The medical stent according to Claim 6,
wherein the engagement portion is a groove portion which is formed at one end of the pressed portion in a width direction of the flap portion so as to penetrate in a thickness direction of the flap portion, and
wherein the filamentous member is arranged in the groove portion.

9. The medical stent according to Claim 1,
wherein the longitudinal member has a tubular shape,
wherein the flap portion is a portion of the side surface of the longitudinal member, and
wherein the side opening is an opening which is formed on the side surface of the longitudinal member by cutting and raising the side surface of the longitudinal member.

10. The medical stent according to Claim 1,
wherein the flap radial-expansion prevention portion is formed to have a belt shape, and
wherein the flap radial-expansion prevention portion is held in the internal space by the insertion member due to a frictional force which is applied between the flap radial-expansion prevention portion passing through the side opening and being arranged in the internal space and the insertion member.

11. The medical stent according to Claim 1,
wherein the flap radial-expansion prevention portion has a first end portion which is connected to the extension portion of the flap portion and a second end portion which is continuous with the first end portion, and
wherein the second end portion of the flap radial-expansion prevention portion at least partially passes through the side opening and the second end portion engages with the insertion member so as to cause the flap portion to maintain the closed state when the flap portion is in the closed state.
